(19) Europäisches Patentamt / European Patent Office / Office européen des brevets

(11) **EP 2 023 268 B1**

(12) **FASCICULE DE BREVET EUROPEEN**

(45) Date de publication et mention
de la délivrance du brevet:
**21.04.2010 Bulletin 2010/16**

(51) Int Cl.:
*G06K 9/00* (2006.01)    *A61B 5/11* (2006.01)
*G06K 9/22* (2006.01)    *G06K 9/68* (2006.01)

(21) Numéro de dépôt: **08159980.5**

(22) Date de dépôt: **09.07.2008**

(54) **Procédé et dispositif de reconnaissance de position ou de mouvement d'un dispositif ou d'un être vivant**

Verfahren und Vorrichtung zur Positions- oder Bewegungserkennung einer Vorrichtung oder eines Lebewesens

Method and device for recognising the position or movement of a device or living being

(84) Etats contractants désignés:
**DE GB IT**

(30) Priorité: **23.07.2007 FR 0756683**

(43) Date de publication de la demande:
**11.02.2009 Bulletin 2009/07**

(73) Titulaire: **Commissariat à l'Energie Atomique
75015 Paris (FR)**

(72) Inventeurs:
• **Bonnet, Stéphane
38170 Seyssinet (FR)**
• **Godin, Christelle
38190 Brignoud (FR)**

(74) Mandataire: **Ilgart, Jean-Christophe et al
Brevalex
3, rue du Docteur Lancereaux
75008 Paris (FR)**

(56) Documents cités:
**WO-A-2005/094676**

• **FAHRENBERG J ET AL: "ASSESSMENT OF POSTURE AND MOTION BY MULTICHANNEL PIEZORESISTIVE ACCELEROMETER RECORDINGS" PSYCHOPHYSIOLOGY, SOCIETY FOR PSYCHOPHYSIOLOGICAL RESEARCH,, US, vol. 34, no. 5, 1997, pages 607-612, XP008041999 ISSN: 0048-5772**
• **VELTINK P H ET AL: "Detection of static and dynamic activities using uniaxial accelerometers" IEEE TRANSACTIONS ON REHABILITATION ENGINEERING, IEEE INC. NEW YORK, US, vol. 4, no. 4, décembre 1996 (1996-12), pages 375-385, XP002315239 ISSN: 1063-6528**
• **MARINS J L ET AL: "An extended kalman filter for quaternion-based orientation estimation using MARG sensors" PROCEEDINGS OF THE 2001 IEEE/RSJ INTERNATIONAL CONFERENCE ON INTELLIGENT ROBOTS AND SYSTEMS. (IROS 2001). MAUI, HAWAII, OCT. 29 - NOV. 3, 2001, IEEE/RSJ INTERNATIONAL CONFERENCE ON INTELLIGENT ROBOTS AND SYSTEMS, NEW YORK, NY : IEEE, US, vol. VOL. 1 OF 4, 29 octobre 2001 (2001-10-29), pages 2003-2011, XP010573412 ISBN: 0-7803-6612-3**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

Le sujet de cette invention est un procédé et un dispositif de reconnaissance de position ou de mouvement d'un dispositif ou d'un être vivant, afin de reconnaître des événements apparaissant pendant le mouvement, et qui peuvent être soit des phases du mouvement soit des états statiques atteints pendant le mouvement, comme des postures physiques d'une personne.

Les applications sont nombreuses. On pourra citer parmi elles la reconnaissance de caractères manuscrits d'après le mouvement d'un stylo ou la surveillance médicale pour repérer des états anormaux d'une personne, susceptibles de signifier un accident ou une crise, telle qu'une crise d'épilepsie.

On s'intéresse particulièrement aux procédés où des capteurs sont fixés au dispositif ou à l'être vivant à observer et en mesurent les mouvements ou la position. Ils comprennent deux genres principaux. Dans le premier d'entre eux, les capteurs sont répartis en grand nombre sur le dispositif ou l'être vivant, et la combinaison de leurs mesures permet de déduire l'information recherchée de façon directe, éventuellement par une représentation graphique. Ces procédés sont simples à exploiter et efficaces entre autres pour déterminer une posture physique, mais l'utilisation de capteurs multiples a un caractère intrusif, désagréable pour une personne porteuse.

Dans un autre genre de procédés, on emploie un seul capteur, ou plus généralement un seul groupe de capteurs, placés ensemble en un même assemblage, ou éventuellement un petit nombre de capteurs répartis sur le dispositif ou l'être vivant. Les mesures prises sont alors trop peu nombreuses pour donner immédiatement l'information souhaitée sur le mouvement (direction, intensité etc.) ou la position (orientation, distance à une référence fixe, etc.) et doivent être interprétées par des règles données arbitrairement ou dégagées à l'issue d'une phase d'apprentissage pendant laquelle le dispositif ou l'être vivant est observé et les mesures prises par les capteurs sont corrélées aux états atteints repérés par l'observateur. L'application des règles permet ensuite de diagnostiquer la réapparition des états repérés pendant la phase d'apprentissage quand des mesures identiques aux états correspondants sont constatées. De telles règles conviennent d'autant mieux que les conditions sont favorables, notamment quand les mouvements sont répétitifs ou que les états atteints sont peu nombreux ou bien caractérisés, ce qui permet des classifications plus faciles. Elles peuvent être mises en échec toutefois, soit en donnant des résultats erronés si elles ont été établies avec imprécision ou s'appliquent à un mouvement inédit, soit en tolérant des indéterminations si elles sont trop peu détaillées. Certaines indéterminations peuvent être intrinsèques au système utilisé : c'est ainsi qu'il est impossible de distinguer une position debout immobile d'une position assise par des capteurs d'orientation ou des accéléromètres fixés sur le torse en considérant seulement l'état statique correspondant. Si ces seconds procédés aux capteurs en nombre réduit conviennent assez bien pour la mesure de l'intensité d'un mouvement (actimétrie), il faudrait les perfectionner pour les rendre plus efficaces pour des études plus fines des mouvements et des positions atteintes afin de reconnaître la posture du porteur puis la nature de son activité ou de son occupation (posturométrie).

Un objet de l'invention est donc de perfectionner ce second genre de procédés, utilisant un petit nombre de capteurs portés à un petit nombre de points de mesure par le dispositif ou l'être vivant, souvent sur un point unique, pour mener à bien une classification d'événements, tels que des postures prises par le porteur quand l'apprentissage a été fait.

La présente invention concerne un procédé de reconnaissance de position ou de mouvement d'un dispositif ou d'un être vivant, comprenant les étapes suivantes: prévoir des premier et second jeux de capteurs sur ou à proximité du dispositif ou de l'être vivant ; relever, pendant une phase d'apprentissage, les mesures prises par les capteurs et appliquer des premières règles de décision prédéfinies au moins aux mesures prises par le premier jeu de capteur pour classer chaque événement identifié à partir des mesures de tout ou partie des capteurs dans une catégorie de position ou de mouvement ; créer une base d'apprentissage, pendant la phase d'apprentissage, répertoriant pour tout ou partie desdits événements leur classification préalablement établie et des caractéristiques issues des mesures du second jeu de capteurs; définir des secondes règles de décision à partir de la base d'apprentissage permettant de classer un événement à partir des mesures issues du second jeu de capteurs ; enlever le premier jeu de et garder seulement le second jeu de capteurs sur le dispositif ou l'être vivant ; et classer la positon ou le mouvement du dispositif ou de l'être, pendant une phase de reconnaissance, en appliquant les secondes règles de décision aux mesures issues du second jeu de capteurs.

Le procédé de la présente invention peut être appliqué à la reconnaissance de postures et de mouvements, les premier et second jeux de capteurs comprenant des capteurs de mouvement tels que des accéléromètres, des magnétomètres et des gyromètres.

Selon un mode de mise en oeuvre du procédé susmentionné, le premier jeu de capteurs comprend des accéléromètres montés sur les cuisses et les jambes de la personne porteuse.

Selon un mode de mise en oeuvre du procédé susmentionné, le second jeu de capteurs comprend un magnétomètre triaxial et un accéléromètre triaxial montés sur une personne porteuse, par exemple sur le torse.

Selon un mode de mise en oeuvre des procédés susmentionnés, les événements classés comprennent des postures debout, assise et couchée ainsi que des transitions entre lesdites postures debout, assise et couchée.

Le procédé de la présente invention peut également être appliqué à la reconnaissance de crises épileptiques, le premier jeu de capteurs comprenant un capteur d'activité électrique du cerveau d'une personne porteuse des capteurs, et le second jeu de capteurs fixés à la personne porteuse comprenant des capteurs de mouvement tels que des accéléromètres,des magnétomètres et des gyromètres.

Le procédé de la présente invention peut également être appliqué à la reconnaissance de crises épileptiques, le premier jeu de capteurs comprenant un enregistreur d'images, et le second jeu de capteurs fixés à une personne porteuse comprenant des capteurs de mouvement tels que des accéléromètres, des magnétomètres et des gyromètres.

En outre, le procédé de la présente invention peut être appliqué à la reconnaissance de caractères manuscrits, le premier jeu de capteurs étant une tablette graphique relié à des moyens d'identification de sigles et le second jeu de capteurs comprenant des capteurs de mouvement montés sur un stylo.

Selon un mode de mise en oeuvre d'un des procédés susmentionnés, le procédé comprend l'entraînement d'un réseau de neurones par la base d'apprentissage pour élaborer les secondes règles de décision.

La présente invention prévoit en outre un dispositif appliquant un des procédés susmentionnés, qui comprend le premier jeu de capteurs et le second jeu de capteurs, et des moyens de traitement des mesures fournies par les capteurs contenant une base d'apprentissage et un classificateur ajustable.

L'invention sera maintenant décrite en liaison aux figures, dont :

- la figure 1 illustre une première application,
- la figure 2 illustre une deuxième application,
- la figure 3 illustre un réseau de neurones,
- la figure 4 est un diagramme de division d'un signal, et
- la figure 5 illustre une troisième application.

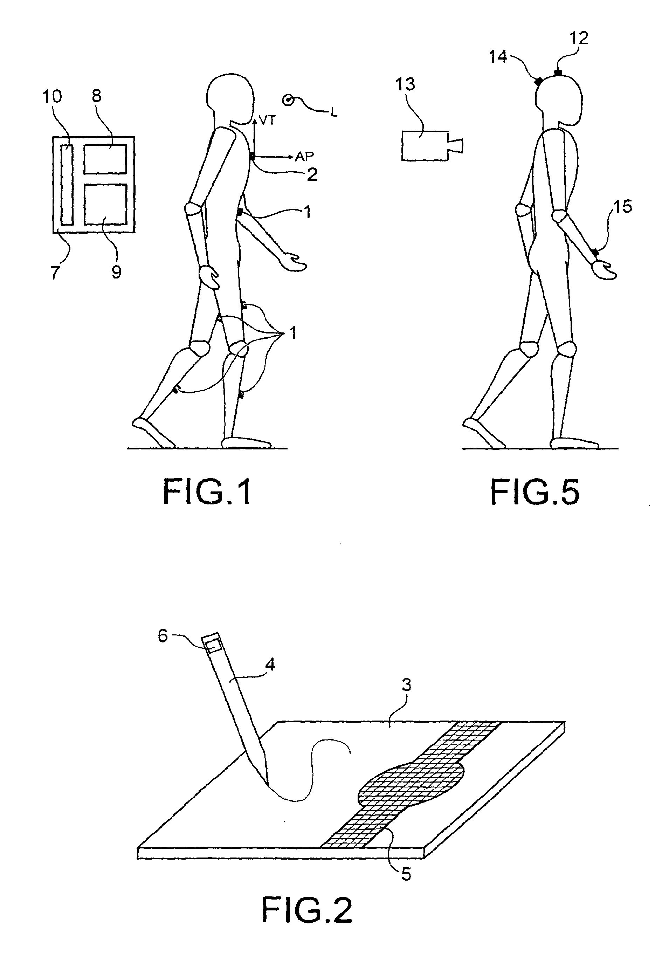

L'exposé porte d'abord sur la figure 1. L'invention repose sur l'emploi de deux jeux de capteurs, dont un second jeu de capteurs ou seconds capteurs 2 concentrés en un seul point de mesure et comprenant, dans la réalisation de la figure 1, un accéléromètre triaxial et un magnétomètre triaxial ou un gyromètre, et un premier jeu de capteurs ou premiers capteurs 1 comprenant notamment des accéléromètres et qui sont répartis sur des points de mesure relativement nombreux. Dans une application courante de l'étude des mouvements de l'être humain, illustrée dans la figure 1, les seconds capteurs 2 peuvent être placés sur le torse, et les premiers capteurs 1 par exemple sur les cuisses et les tibias, ou sur d'autres régions du torse.

Dans cette réalisation comme dans les autres, le système comprend un ordinateur 7 recueillant les mesures de tous les capteurs 1 et 2 et comprenant une base d'apprentissage 8 ainsi qu'un classificateur 9 et un élément d'exploitation 10 des résultats donnés par le classificateur 9 de nature adaptée aux fins du procédé (mémoire enregistreuse de déplacements et d'états, interface graphique, système d'alarme, etc).

Le procédé de reconnaissance selon la présente invention se décompose en deux grandes phases : une phase d'apprentissage et une phase de reconnaissance. Lors de la phase d'apprentissage, on procède à un suivi et à une analyse des mouvements effectués par la personne étudiée sur laquelle sont placés les premier et second jeux de capteurs 1 et 2. Lors de la phase de reconnaissance, on définit la position ou le type de mouvement effectué par la personne en utilisant uniquement le second jeu de capteurs, le premier jeu de capteurs ayant été retiré.

Lors de la phase d'apprentissage, les mesures fournies par les différents capteurs 1 et 2 sont transmises à l'ordinateur 7. Le classificateur 9 utilise alors tout ou partie des mesures fournies par les capteurs 1 et 2 pour déterminer avec une confiance élevée les postures ou les mouvements du porteur des capteurs 1 et 2. Pour ce faire, le classificateur 9 utilise des premières règles de décision préalablement définies. On pourra par exemple utiliser des premières règles de décision classiques utilisant uniquement les mesures fournies par les différents accéléromètres des premiers et seconds jeux de capteurs 1 et 2. On notera que l'utilisation des mesures fournies par les seconds capteurs est facultative pour réaliser la classification.

Pour des exemples de premières règles de décision, on pourra se référer aux articles suivants :

- Article de P.H. Veltink, H.B.J Bussmann, W. de Vries, W.L.J. Martens et R.C. van Lummel, intitulé "Detection of static and dynamic activities using uniaxial accelerometers", IEEE Trans. Rehab. Eng., vol 4, n° 4, pages 375-385, de décembre 1996 ;
- Article de G. M. Lyons, K.M. Culhane, D. Hilton, P.A. Grace and D. Lyons, intitulé "A description of an accelerometer-based mobility monitoring technique", Medical Engineering and Physics, vol. 27, pages 497-504, de 2005 ;
- brevet US numéro 6,834,436 de Microstrain Inc, intitulé "Posture and body movement measuring system".

Dans l'exemple de la figure 1, le classificateur sert à attribuer une classification (par exemple Assis, Debout, Couché, Transition Assis-couché, Transition Assis-debout, etc...) à chaque événement détecté à partir des mesures des capteurs. Pour déterminer ces événements successifs et attribuer une classification, le classificateur peut utiliser directement les mesures des capteurs ou calculer les valeurs de différents paramètres (valeur moyenne, énergie ...) à partir des mesures des capteurs.

On utilisera de préférence uniquement les mesures des seconds capteurs pour identifier les événements successifs, c'est-à-dire pour "découper" temporellement les signaux reçus en une suite séquentielle d'événements. Une fois cette suite séquentielle d'événements définie, on utilise les mesures des premiers capteurs et éventuellement de tout ou partie des seconds capteurs pour attribuer une classification à chaque événement préalablement identifié. Un exemple de mode de mise en oeuvre d'un tel procédé d'identification et de classification d'événements est donné dans la suite de la description.

Au fur et à mesure des classifications d'événements réalisées par le classificateur, on enrichit, automatiquement, la base d'apprentissage 8 avec de nouvelles données. Ainsi, pour chaque classification d'un événement réalisée par le classificateur 9 on ajoute une donnée dans la base d'apprentissage. Chaque nouvelle donnée comprend des "caractéristiques" obtenues à partir des mesures fournies par le second jeu de capteurs 2 et la classification correspondante indiquée par le classificateur 9. Le terme "caractéristiques" signifie une ou plusieurs valeurs correspondant soit à une mesure d'un des capteurs du jeu de capteurs 2 soit à une valeur calculée, définie, à partir de tout ou partie des mesures fournies par le second jeu de capteur 2. Des exemples de caractéristiques pouvant être utilisées dans le cadre d'une reconnaissance de posture ou de mouvement d'une personne sont la durée de l'événement considéré, une inclinaison de la personne dans un plan donné, des valeurs "de lacet" du torse correspondant à une orientation moyenne par rapport au nord magnétique et à un écart type par rapport à cette orientation moyenne d'un signal d'un magnétomètre sur la durée de l'événement considéré, et l'énergie d'un signal d'accélération.

Cette base d'apprentissage permet ensuite d'entraîner un classificateur tel qu'un réseau de neurones par un procédé d'apprentissage supervisé et de déterminer des secondes règles optimales de classification de postures à appliquer aux signaux des seconds capteurs 2.

La phase d'apprentissage peut être arrêtée avec un nombre de données suffisant, lorsqu'on peut estimer que les secondes règles ont été établies avec une précision suffisante. Il est possible de le faire en les mettant à l'épreuve sur une portion de la base d'apprentissage, appelée base de validation et qui n'est pas utilisée pour construire les secondes règles.

On remarquera que les secondes règles de classification, étant implicites et ajustées de façon souple, sont adaptées à exprimer les particularités des mouvements du porteur et de son environnement.

Dans les périodes de reconnaissance qui suivent la période d'apprentissage, les premiers capteurs 1 sont retirés et le porteur garde seulement les seconds capteurs 2. Leurs signaux sont extraits et transmis au classificateur 9, qui applique les secondes règles de décision.

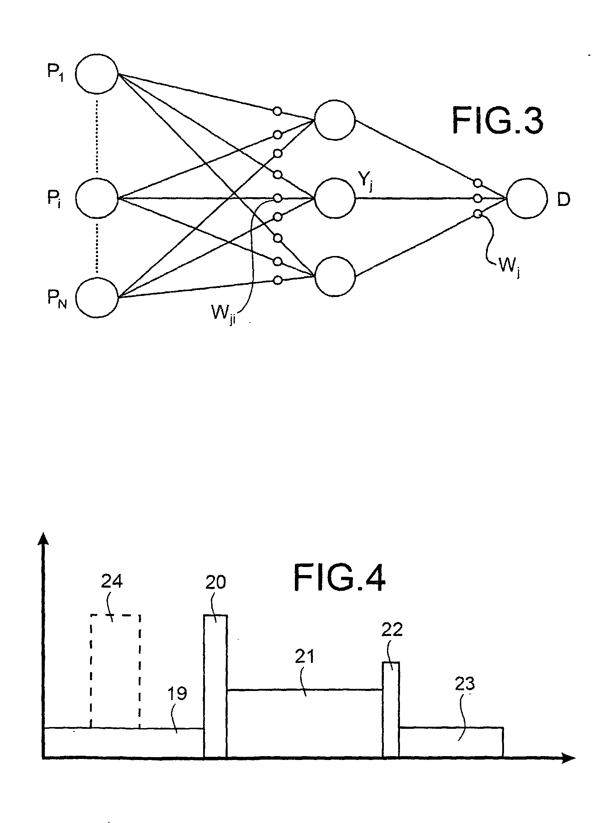

Un exemple de détermination d'une séquence d'événements pouvant être utilisée dans le cadre d'une reconnaissance de posture ou de mouvement d'une personne ainsi qu'un exemple de classification détaillée sont donnés ci-après. A partir des signaux issus des capteurs 2, on évalue une caractéristique appelée énergie d'activité dont un exemple est représenté en figure 4. Afin de définir une séquence d'événements, on applique des règles de décision de "préclassification" afin de distinguer entre certains genres d'activité de la personne et certaines de ses postures. Dans le diagramme de la figure 4, exprimé en énergie d'activité en fonction du temps, on trouve successivement un état debout, une transition debout/assis, un état assis, une transition assis/debout et un état debout aux états 19 à 23. Les états de transition correspondent toujours à des activités fortes et à des pics d'énergie, et des états de position stable sont souvent plus longs et moins actifs, mais des exceptions existent, comme la course, pendant laquelle le porteur en position debout stable aura une grande énergie. De plus, l'énergie peut varier fortement pendant un même état. Une période de course 24 pendant le premier état debout 19 est ainsi représentée. Il n'est donc pas possible de déduire directement la succession des états d'un diagramme tel que celui de la figure 5, mais il permet d'effectuer une division rationnelle en segments de durées inégales de caractéristiques homogènes.

Ainsi, l'enregistrement de la caractéristique "activité d'énergie" est découpé en portions ou segments temporels selon un ensemble de plusieurs critères. On accède ainsi à une alternance de zones homogènes au sens des postures (appelées "O-segments") et de zones actives ("1-segments") qui représentent potentiellement des transferts posturaux. Chaque segment temporel désigne un événement.

On effectue ensuite une classification de chaque événement en utilisant les mesures des capteurs 1 et éventuellement de tout ou partie des capteurs 2. A titre indicatif, les classes de posture statique ou dynamique peuvent être au nombre de neuf : trois états stables (assis, debout et couché), et six états de transfert d'un à un autre des états stables précédents (debout vers assis, assis vers debout, debout vers couché, couché vers debout, assis vers couché et couché vers assis).

On décrira ci-après, comme exemple de classificateur apte à appliquer les secondes règles de décision, l'utilisation d'un réseau de neurones à la figure 3. Le réseau comprend une couche de neurones d'entrée P1, P2, ..., Pn pour recevoir les valeurs des caractéristiques des signaux des seconds capteurs 2 sous forme numérique.

La décision de classification dans une classe déterminée est donnée, aussi sous forme numérique, par le contenu d'un neurone de sortie D. Le réseau de neurones comprend au moins une couche cachée de neurones $y_j$ qui

effectuent des combinaisons numériques des paramètres et les transmettent à la couche suivante du réseau, ici immédiatement au neurone de sortie D. Des poids numériques w sont appliqués aux valeurs transmises avant qu'elles n'atteignent les neurones de la couche cachée ou le neurone de sortie. <u>Ici</u>, les neurones de la couche cachée effectuent une activation en tangente hyperbolique et le neurone de sortie effectue une activation linéaire. La fonction de décision peut alors être écrite d'après la formule (1)

$$(1) \qquad D_W(\bar{P}) = \text{sgn}\left( \sum_j w_j \tanh\left( \sum_i w_{ji} p_i + w_{oj} \right) + w_o \right)$$

où sgn est la fonction signe, tanh la fonction tangente hyperbolique, $W_j$ le poids reliant la sortie du neurone $y_j$ au neurone de sortie D, $w_0$ et $w_{0j}$ des poids particuliers appelés biais ou seuils reliant un neurone fictif de sortie de valeur égale à 1 au neurone de sortie (pour $w_0$) ou aux neurones $y_j$ de la couche cachée (pour $w_{0j}$) et $w_{ji}$ les poids reliant les neurones d'entrée $P_i$ aux neurones de la couche cachée $y_j$.

Les réseaux de neurones permettent, sous réserve de bien choisir le nombre de neurones dans la couche cachée ou les couches cachées, d'approcher n'importe quelle fonction une fois que les bons poids ont été trouvés. L'élaboration des secondes règles de décision revient donc à ajuster les poids w de manière à faire coïncider, pour chaque exemple ou presque de la base d'apprentissage, le résultat de la décision par le réseau de neurones et le résultat, connu par les premières règles, de la classification. Dans la phase d'apprentissage, un ordinateur associé au système ajuste les poids de manière que le plus grand nombre d'événements de la base d'apprentissage soit bien évalué par les secondes règles de décision. On remarquera que cet ajustement des poids permet de modeler automatiquement les secondes règles de décision pour les perfectionner.

D'autres genres de classificateurs numériques sont connus dans l'art : séparateurs linéaires, à vaste marge, arbres de décision, SVM (machine à support de vecteurs) ; l'invention s'applique aussi à eux.

Les caractéristiques servant à régler le classificateur sont calculés directement sur les signaux acquis par les seconds capteurs 2 ou sur les signaux dérivés (angle d'inclinaison, angle de lacet, index d'activité, ...). Les caractéristiques calculées sont par exemple, pour chaque segment

- statistiques sur les signaux,
- durée,
- contenu fréquentiel (répartition des pics d'énergie au domaine fréquentiel),
- voire le segment lui-même (signature). D'autres types de capteurs peuvent être employés, comme des gyromètres ou des capteurs d'activité physiologique du porteur. Le premier jeu de capteurs peut être placé sur un point de mesure différent, comme le dos ou la ceinture, voire réparti en un petit nombre de points de mesure sur le porteur. Les états reconnus par le système peuvent être non seulement des postures statiques mais les transferts d'une posture à une autre.

Une autre application concrète de la présente invention consiste en la surveillance de patients épileptiques (figure 5) au moyen de dispositifs de surveillance simples tels que des capteurs de mouvement.

Lors d'une période d'apprentissage, on place sur ou à proximité du patient un premier jeu de capteurs. Le premier jeu de capteurs peut consister en un capteur d'activité électrique du cerveau 12 du patient et/ou en une caméra vidéo 13 qui enregistre son activité. Un capteur d'activité électrique du cerveau 12 consiste en un jeu d'électrodes implantées ou posées en surface du cerveau et reliées à un système d'enregistrement de l'activité électrique d'une partie du cerveau. Lors de cette phase d'apprentissage, on place également sur le patient un second jeu de capteurs 2 destinés à relever les mouvements d'une partie du corps du patient. Ce second jeu de capteurs peut être constitué d'accéléromètres, de magnétomètres et/ou de gyromètres 14 ou 15 portés sur la tête ou les poignets du patient. Ce second jeu est destiné à détecter des mouvements caractéristiques d'une crise tels que des tremblements, des rotations lentes de la tête ou du bras, etc ...

Les capteurs 1 et 2 sont reliés à un ordinateur tel que celui représenté en figure 1 qui comprend une base d'apprentissage 8, un classificateur 9 et un élément d'exploitation 10.

Lors de cette phase d'apprentissage, on utilise la caméra vidéo 13 ou le capteur d'activité électrique 12 pour déterminer la présence ou non d'une crise. On cherche au final à identifier deux classes d'événements, à savoir les classes "crise" et "non-crise".

On construit la base d'apprentissage de la façon suivante. On identifie une suite d'événements à partir des mesures issues des capteurs de mouvement 14, 15 (second jeu de capteurs). Par événement, on entend une rotation de la tête, un tremblement du poignet, etc... A tout ou partie de ces événements identifiés, on associe une classification "crise" ou "non-crise" donnée, cette classification étant réalisée par le classificateur 9 à partir des signaux issus du premier jeu de capteurs 12 et/ou 13. Chaque association événement/classification constitue une nouvelle donnée qui est enregistrée dans la base d'apprentissage 8.

A l'issue de cette phase d'apprentissage, ou au fur et à mesure de cette dernière, on définit, à partir de la base d'apprentissage, des secondes règles de classification qui seront utilisées par le classificateur 9 lors de la phase d'évaluation (phase de surveillance). Le classificateur susceptible de mettre en oeuvre ces secondes règles de classification peut là aussi être un réseau de neurones.

Les premiers capteurs sont ensuite retirés et les crises suivantes seront diagnostiquées au moyen des seconds capteurs seuls, les secondes règles de décision permettant de distinguer l'activité normale de l'activité épileptique.

Contrairement aux dispositifs de surveillance de l'art antérieur qui utilisent une caméra vidéo en permanence ou un capteur d'activité électrique du cerveau, la présente invention permet de détecter des crises épileptiques au moyen de dispositifs peu intrusifs et qui peuvent être utilisés partout et non uniquement dans une pièce équipée d'une caméra vidéo. On pourra se référer aux articles suivants. Pour l'utilisation de capteurs d'activité électrique on pourra se référer à l'article de N.C. Bhavaraju, M.G. Frei et I Osorio, intitulé "Analog Seizure Detection and Performance Evaluation", IEEE Trans. On Biomedical Eng., vol. 53, n° 2, de février 2006. Pour l'utilisation d'une caméra vidéo, on pourra se référer à l'article de Nicolaos B. Karayiannis, et autres, intitulé "Automated Détection of Videotaped Neonatal Seizures of Epileptic Origin", Epilepsia, 47(6):966-980, de 2006.

Un autre mode de mise en oeuvre de l'invention est maintenant décrit au moyen de la figure 2. Il s'agit de la reconnaissance de caractères manuscrits à mesure qu'ils sont écrits. Lors d'une phase d'apprentissage, on dispose d'une tablette graphique 3 (existant sous le nom de Flybook) équipée de capteurs d'écriture sous sa surface, composant un premier jeu de capteurs 5, et d'un algorithme de reconnaissance des caractères qui sont écrits sur elle, cet algorithme mettant en oeuvre des premières règles de décision. L'inscription sur la tablette graphique 3 se fait avec un stylo 4 équipé d'un second jeu de capteurs 6, qui peuvent consister en des accéléromètres, magnétomètres, gyromètres ou capteurs de force. La création de la base d'apprentissage consiste, pour un certain nombre de lettres ou de mots écrits, à les reconnaître par la tablette graphique 3, et à les corréler aux mesures prises par le second jeu de capteurs 6 sur le stylo 4. Dans la phase suivante de reconnaissance, où la tablette graphique 3 est retirée, les mesures du second jeu de capteurs 6 servent à identifier directement les caractères tracés sur un support quelconque.

Ainsi qu'on l'a vu, si les capteurs les plus fréquents sont des capteurs de contact, fixés au dispositif ou à l'être vivant qui est l'objet du procédé, l'invention n'est pas exclusive de capteurs à distance placés devant lui, qui enregistrent son mouvement ou d'autres paramètres par l'intermédiaire d'une image, d'une distance ou d'autres paramètres.

Par ailleurs, l'ordinateur utilisé dans les exemples de mise en oeuvre de l'invention susmentionnés peut être remplacé par tout type de moyens de traitement des mesures fournis par tout ou partie des capteurs. On pourra par exemple utiliser un ordinateur lors de la phase d'apprentissage, puis uniquement un dispositif de traitement portatif placé sur le dispositif ou l'être vivant dont on souhaite suivre les positions ou les mouvements. De même, la liaison entre les capteurs et les moyens de traitement peut être filaire ou hertzienne.

## Revendications

1. Procédé de reconnaissance de position ou de mouvement d'un dispositif ou d'un être vivant, comprenant les étapes suivantes:

   - prévoir des premier et second jeux de capteurs (1, 2, 5, 6, 12, 13, 14, 15) sur ou à proximité du dispositif ou de l'être vivant ;
   - relever, pendant une phase d'apprentissage, les mesures prises par les capteurs et appliquer des premières règles de décision prédéfinies au moins aux mesures prises par le premier jeu de capteurs pour classer chaque événement identifié à partir des mesures de tout ou partie des capteurs dans une catégorie de position ou de mouvement ;
   - créer une base d'apprentissage, pendant la phase d'apprentissage, répertoriant pour tout ou partie desdits événements leur classification préalablement établie et des caractéristiques issues des mesures du second jeu de capteurs;
   - définir des secondes règles de décision à partir de la base d'apprentissage permettant de classer un événement à partir des mesures issues du second jeu de capteurs ;
   - enlever le premier jeu de capteurs (1, 6, 12, 13) et garder seulement le second jeu de capteurs (2, 5, 14, 15) sur le dispositif ou l'être vivant ; et
   - classer la positon ou le mouvement du dispositif ou de l'être vivant, pendant une phase de reconnaissance, en appliquant les secondes règles de décision aux mesures issues du second jeu de capteurs.

2. Procédé selon la revendication 1, **caractérisé en ce qu'**il est appliqué à la reconnaissance de postures et de mouvements, les premier et second jeux de capteurs comprenant des capteurs de mouvement tels que des accéléromètres, des magnétomètres et des gyromètres.

3. Procédé selon la revendication 2, **caractérisé en ce que** le second jeu de capteurs comprend un magnétomètre triaxial et un accéléromètre triaxial montés sur une personne porteuse.

4. Procédé selon la revendication 3, **caractérisé en ce que** le premier jeu de capteurs comprend des accéléromètres montés sur les cuisses et les jambes de la personne porteuse.

5. Procédé selon la revendication 2, 3 ou 4, **caractérisé en ce que** les événements classés comprennent des postures debout, assise et couchée ainsi que des transitions entre lesdites postures debout, assise et couchée.

6. Procédé selon la revendication 1, **caractérisé en ce qu'**il est appliqué à la reconnaissance de crises épileptiques, le premier jeu de capteurs comprenant un capteur d'activité électrique du cerveau d'une personne porteuse des capteurs, et le second jeu de capteurs fixés à la personne porteuse comprenant des capteurs de mouvement tels que des accéléromètres, des magnétomètres et des gyromètres.

7. Procédé selon la revendication 1, **caractérisé en ce qu'**il est appliqué à la reconnaissance de crises épileptiques, le premier jeu de capteurs comprenant un enregistreur d'images, et le second jeu de capteurs fixés à une personne porteuse comprenant des capteurs de mouvement tels que des accéléromètres, des magnétomètres et des gyromètres.

8. Procédé selon la revendication 1, **caractérisé en ce qu'**il est appliqué à la reconnaissance de caractères manuscrits, le premier jeu de capteurs étant une tablette graphique reliée à des moyens d'identification de signes et le second jeu de capteurs comprenant des capteurs de mouvement montés sur un stylo.

9. Procédé selon l'une quelconque des revendications précédentes, **caractérisé en ce qu'**il comprend l'entraînement d'un réseau de neurones par la base d'apprentissage pour élaborer les secondes règles de décision.

10. Dispositif adapté à appliquer le procédé selon l'une quelconque des revendications précédentes, **caractérisé en ce qu'**il comprend le premier jeu de capteurs et le second jeu de capteurs, et des moyens de traitement des mesures fournies par les capteurs contenant une base d'apprentissage et un classificateur ajustable.

## Claims

1. Method for the recognition of the position or movement of a device or a live being, comprising the following steps:

   - providing first and second sets of sensors (1, 2, 5, 6, 12, 13, 14, 15) on or close to the device or live being;
   - measuring, during a learning phase, the readings recorded by the sensors and applying first predefined decisional rules at least to the measurements taken by the first set of sensors to classify each event identified using the measurements of all or part of the sensors into a position or movement category;
   - creating a learning base, during the learning phase, identifying for all or part of said events their pre-established classification and the characteristics from the measurements of the second set of sensors;
   - defining second decisional rules based on the learning base permitting an event to be classified based on measurements taken by the second set of sensors;
   - removing the first set of sensors (1, 6, 12, 13) and only keeping the second set of sensors (2, 5, 14, 15) on the device or the live being; and
   - classifying the position or the movement of the device or live being, in a recognition phase, by applying the second decisional rules to the measurements made by the second set of sensors.

2. Method according to claim 1, **characterised in that** it is applied to the recognition of postures and movements, wherein the first and second sets of sensors comprise movement sensors such as accelerometers, magnetometers and gyrometers.

3. Method according to claim 2, **characterised in that** the second set of sensors comprises a tri-axial magnetometer and a tri-axial accelerometer fitted to a wearer.

**4.** Method according to claim 3, **characterised in that** the first set of sensors comprises accelerometers fitted to the thighs and legs of the wearer.

**5.** Method according to claim 2, **characterised in that** the classified events comprise standing, seated and lying down postures as well as transitions between said standing, seated and lying down postures.

**6.** Method according to claim 1, **characterised in that** it is applied to the recognition of epileptic fits, wherein the first set of sensors comprises a sensor for the electrical activity of the brain of a live being wearing the sensors, and the second set of sensors fitted to the wearer comprises movement sensors such as accelerometers, magnetometers and gyrometers.

**7.** Method according to claim 1, **characterised in that** it is applied to the recognition of epileptic fits, wherein the first set of sensors comprises an image recording device, and the second set of sensors fitted to the wearer comprises movement sensors such as accelerometers, magnetometers and gyrometers.

**8.** Method according to claim 1, **characterised in that** it is applied to the recognition of handwritten characters, wherein the first set of sensors is a graphic pad connected to sign identification means and the second set of sensors comprises movement sensors mounted on a pen.

**9.** Method according to claim 1, **characterised in that** it comprises a training of a neurone system with the learning base to draft the second decisional rules.

**10.** Device suitable for the application of a method according to any one of the previous claims, **characterised in that** it comprises the first set of sensors and the second set of sensors, and means of processing the measurements provided by the sensors containing a learning base and an adjustable classifier.

**Patentansprüche**

**1.** Verfahren zur Untersuchung und Erkennung von Lage/Stellung oder Bewegung einer Vorrichtung oder eines Lebewesens, wobei das Verfahren die folgenden Schritte umfasst:

- es werden erste und zweite Sätze von Messfühlern (1, 2, 5, 6, 12, 13, 14, 15) an/auf oder nahe der Vorrichtung oder dem Lebewesen vorgesehen;
- während einer Lernphase werden die durch die Messfühler ermittelten Messwerte erhoben und erste vorbestimmte Entscheidungsregeln wenigstens auf die durch den ersten Messfühlersatz ermittelten Messwerte angewandt, um jeweils jedes auf der Grundlage der Messwerte aller oder eines Teils der Meßfühler identifizierte Ereignis in einer Kategorie von Stellung/Lage oder Bewegung zu klassifizieren;
- während der Lernphase wird eine Lernbasis geschaffen, indem für jedes oder einen Teil der genannten Ereignis (se) ihre zuvor festgestellte Klassifikation sowie aus den Messwerten des zweiten Messfühlersatzes gewonnene charakteristische Eigenschaften verzeichnet werden;
- auf der Grundlage der Lernbasis werden zweite Entscheidungsregeln definiert, welche die Klassifizierung eines Ereignisses auf der Grundlage der von dem zweiten Messfühlersatz gewonnenen Messwerte gestattet;
- unter Weglassung des ersten Satzes von Messfühlern (1, 6, 12, 13) wird nur der zweite Satz von Messfühlern (2, 5, 14, 15) an/auf der Vorrichtung oder dem Lebewesen beibehalten;
- während einer Erkundungs- bzw. Erkennungsphase wird durch Anwendung der zweiten Entscheidungsregeln auf die von dem zweiten Messfühlersatz herrührenden Messwerte die Stellung/Lage bzw. die Bewegung der Vorrichtung oder des Lebewesens klassifiziert.

**2.** Verfahren nach Anspruch 1, **dadurch gekennzeichnet, dass** das Verfahren auf die Erkundung bzw. Erkennung von Haltungen oder Bewegungen angewandt wird, wobei die ersten und zweiten Messfühlersätze Bewegungs-Messfühler wie beispielsweise Accelerometer (Beschleunigungsmesser), Magnetometer und Gyrometer aufweisen.

**3.** Verfahren nach Anspruch 2, **dadurch gekennzeichnet, dass** der zweite Messfühlersatz ein Triaxial-Magnetometer und ein Triaxial-Accelerometer jeweils in Anbringung auf einer sie tragenden Person umfasst.

**4.** Verfahren nach Anspruch 3, **dadurch gekennzeichnet, dass** der erste Messfühlersatz Accelerometer aufweist, die an den Oberschenkeln und an den Beinen der Trägerperson angebracht sind.

**5.** Verfahren nach Anspruch 2, 3 oder 4, **dadurch gekennzeichnet, dass** die klassifizierten Ereignisse aufrechtstehende, sitzende und liegende Haltungen sowie Übergänge zwischen den genannten Haltungen aufrechtstehend, sitzend und liegend umfassen.

**6.** Verfahren nach Anspruch 1, **dadurch gekennzeichnet, dass** das Verfahren auf die Untersuchung bzw. Erkennung epileptischer Krisen angewandt wird, wobei der erste Messfühlersatz einen Messfühler für die elektrische Energie des Gehirns einer Messfühler tragenden Person umfasst und dass der zweite Satz von an der Trägerperson befestigten Messfühlern Bewegungsmessfühler wie beispielsweise Accelerometer, Magnetometer und Gyrometer umfasst.

**7.** Verfahren nach Anspruch 1, **dadurch gekennzeichnet, dass** das Verfahren auf die Untersuchung bzw. Erkennung epileptischer Krisen angewandt wird, wobei der erste Messfühlersatz eine Bildaufzeichnungsvorrichtung umfasst und wobei der zweite, an einer Trägerperson befestigte Messfühlersatz BewegungsMessfühler wie beispielsweise Accelerometer, Magnetometer und Gyrometer aufweist.

**8.** Verfahren nach Anspruch 1, **dadurch gekennzeichnet, dass** es auf die Untersuchung bzw. Erkennung von handschriftlichen Buchstaben angewandt wird, wobei der erste Messfühlersatz eine mit Mitteln zur Zeichenidentifizierung verbundene Graphiktafel umfasst und wobei der zweite Messfühlersatz an einem Schreibstift angeordnete BewegungsMessfühler umfasst.

**9.** Verfahren nach einem der vorhergehenden Ansprüche, **dadurch gekennzeichnet, dass** das Verfahren das Training eines Neuronennetzes durch die Lernbasis, zur Ausarbeitung der zweiten Entscheidungsregel umfasst.

**10.** Zur Anwendung des Verfahrens nach einem der vorhergehenden Ansprüche angepasste Vorrichtung, **dadurch gekennzeichnet, dass** die Vorrichtung den ersten und den zweiten Satz von Messfühlern umfasst, sowie Mittel zur Verarbeitung der von den Messfühlern gelieferten Messwerte umfasst, wobei diese Verarbeitungsmittel eine Lernbasis sowie einen einstellbaren Klassifikator umfassen.

FIG.1

FIG.5

FIG.2

FIG.3

$P_1$

$P_i$

$P_N$

$W_{ji}$

$Y_j$

$W_j$

D

FIG.4

24

20

21

22

19

23

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6834436 B

**Littérature non-brevet citée dans la description**

- **P.H. VELTINK ; H.B.J BUSSMANN ; W. DE VRIES ; W.L.J. MARTENS ; R.C. VAN LUMMEL.** Detection of static and dynamic activities using uniaxial accelerometers. *IEEE Trans. Rehab. Eng.,* vol. 4, 375-385
- **G. M. LYONS ; K.M. CULHANE ; D. HILTON ; P.A. GRACE ; D. LYONS.** A description of an accelerometer-based mobility monitoring technique. *Medical Engineering and Physics,* 2005, vol. 27, 497-504

- **N.C. BHAVARAJU ; M.G. FREI ; I OSORIO.** Analog Seizure Detection and Performance Evaluation. *IEEE Trans. On Biomedical Eng.,* Février 2006, vol. 53 (2
- **NICOLAOS B. KARAYIANNIS.** Automated Détection of Videotaped Neonatal Seizures of Epileptic Origin. *Epilepsia,* 2006, vol. 47 (6), 966-980